# EUROPEAN PATENT APPLICATION

(11) **EP 4 844 555 A2**
(43) Date of publication of application: **25.11.2026**
(21) Application number: 26205264.0
(22) Date of filing: 27.09.2017
(51) Int. Cl.: G06F 3/04847

(54) **PERSONALIZING PRESET MEAL SIZES IN INSULIN DELIVERY SYSTEM**

(30) Priority: 27.09.2016 US 201662400366 P
(62) Document number(s) of the earlier application(s) in accordance with Art. 76 EPC: 24153294.4 / 4 386 533; 17857362.2 / 3 518 763
(71) Applicant: Bigfoot Biomedical, Inc., Milpitas, CA 95035 (US)
(72) Inventor: DESBOROUGH, Lane, Milpitas, 95035 (US); MAZLISH, Bryan, Milpitas, 95035 (US); BOISSIER, Jeff, Milpitas, 95035 (US); KABEL-ECKES, Sabine, Milpitas, 95035 (US); NAYLOR, Ross, Milpitas, 95035 (US); BOCHENKO, Andrew, Milpitas, 95035 (US)
(74) Representative: Murgitroyd & Company

(57) **Abstract**

A method may include displaying at least three icons on a user interface of a mobile device, including a first icon associated with the at least three icons associated with a first carbohydrate level, a second icon associated with the at least three icons associated with a second carbohydrate level, and a third icon associated with the at least three icons associated with a third carbohydrate level. The method may also include receiving a user selection of one of the three icons through the user interface of the mobile device, and determining an insulin bolus level from the user selection. The method may also include communicating the insulin bolus level to an insulin delivery device

## Description

### CROSS-REFERENCE TO RELATED APPLICATIONS

A claim for benefit of priority to the September 27, 2016 filing date of the U.S. Patent Provisional Application No. 62/400,366, titled PERSONALIZING PRESET MEAL SIZES IN INSULIN DELIVERY SYSTEM (the '366 Provisional Application), is hereby made pursuant to 35 U.S.C. § 119(e). The entire disclosure of the '366 Provisional Application is hereby incorporated by reference.

### TECHNICAL FIELD

This invention relates to the perso nalizing of preset meal sizes for a user to enter meal data into an insulin delivery system. For example, one or more meal icons can be personalized to describe an amount or range of amounts of carbohydrates for an insulin bolus calculation.

### BACKGROUND

Diabetes mellitus is a chronic metabolic disorder caused by an inability of a person's pancreas to produce sufficient amounts of the hormone, insulin, such that the person's metabolism is unable to provide for the proper absorption of sugar and starch. This failure leads to hyperglycemia, i.e. the presence of an excessive amount of analyte, such as glucose, within the blood plasma. Persistent hyperglycemia has been associated with a variety of serious symptoms and life threatening long-term complications such as dehydration, ketoacidosis, diabetic coma, cardiovascular diseases, chronic renal failure, retinal damage and nerve damages with the risk of amputation of extremities. Self-monitoring of blood glucose and the self-administration of insulin is the typical method for treating diabetes. In order to assist with this self-treatment, many diabetes-related devices (e.g., blood glucose meters, insulin pumps, etc.) are equipped with insulin bolus calculators that have the user input a number of carbohydrates consumed (or about to be consumed) and the bolus calculator outputs a recommended size for the insulin bolus dosage. Although bolus calculators remove some of the calculations that need to be made by the user in determining an appropriate insulin bolus dosage, bolus calculators still burden the user with the mental task of determining the number of carbohydrates in their meal. Accordingly, there is a need for methods, systems, and devices that further reduce the cognitive burden on the user while improving the accuracy of a recommended insulin bolus dosage.

### SUMMARY

Some embodiments include an insulin delivery system that includes an insulin delivery device adapted to receive insulin and deliver insulin subcutaneously; a user interface in communication with the insulin delivery device and adapted to send the insulin delivery device bolus insulin instructions, the user interface including multiple user-selectable icons or buttons each representing an amount of carbohydrates; memory to store one or more user-specific dosage parameter; and a processor in communication with the memory and adapted to receive blood glucose data, the processor being adapted to determine the amount of carbohydrates associated with each of the user-selectable icons or buttons based on at least one of the user-specific dosage parameters, the processor further being adapted to update the amount of carbohydrates associated with each of the user-selectable icons or buttons based upon the blood glucose data.

In some embodiments, the user-selectable icons or buttons each represent an amount of carbohydrates in 5 gram or 10 gram increments. In some embodiments, the amount of carbohydrates represented by each of the icons is determined based on an insulin Sensitivity Factor (ISF), a Carb Ratio (CR), a body weight, an age, a total daily basal dose (TDBD), and/or a combination thereof of a person with diabetes (PWD).

In some embodiments, the processor is further configured to determine an insulin delivery amount based on an amount of carbohydrates associated with a selected one of the user selectable icons or buttons and/or the blood glucose data.

In some embodiments, the user-selectable icons or buttons each represent an amount of carbohydrates rounded to the nearest 5 grams.

In some embodiments, the insulin delivery system may include a glucose monitor adapted to monitor the glucose level of a person with diabetes and provide blood glucose data to the processor.

In some embodiments, the amount of carbohydrates associated with each of the user-selectable icons is determined from postprandial blood glucose data.

Some embodiments may include a method that includes displaying at least three icons on a user interface of a mobile device, including a first icon associated with the at least three icons associated with a first carbohydrate level, a second icon associated with the at least three icons associated with a second carbohydrate level, and a third icon associated with the at least three icons associated with a third carbohydrate level; receiving a user selection of one of the three icons through the user interface of the mobile device; determining an insulin bolus level from the user selection; and communicating the insulin bolus level to an insulin delivery device.

In some embodiments, the method may include receiving blood glucose data; wherein the insulin bolus level is determined in part from the blood glucose data.

In some embodiments, adjusting the first carbohydrate level, the second carbohydrate level, and the third carbohydrate level based on an insulin Sensitivity Factor (ISF), a Carb Ratio (CR), a body weight, an age, a total daily basal dose (TDBD) (which can be characterized as a rate), and/or a combination thereof of a person with diabetes (PWD).

In some embodiments, the insulin bolus level is communicated to the insulin delivery device in response to a user selection indicating delivery of the insulin, wherein the user selection includes a fail safe procedure.

In some embodiments, the insulin bolus level is communicated to the insulin delivery device in response to a user selection indicating delivery of the insulin, wherein the user selection includes a plurality of taps or gestures from a user.

In some embodiments, the first carbohydrate level is rounded to the nearest 5 grams, the second carbohydrate level is rounded to the nearest 5 grams, and the third carbohydrate level is rounded to the nearest 5 grams.

In some embodiments, the method may include receiving postprandial blood glucose data; and adjusting at least one of the first carbohydrate level, the second carbohydrate level, and the third carbohydrate level based on the postprandial blood glucose data.

In some embodiments, the method may include the insulin bolus level is determined from one or more of the following factors the number of carbohydrates divided by the PWD's carbohydrate-to-insulin ratio, a difference between the current blood glucose level and a target blood glucose level divided by the PWD's insulin sensitivity factor, a reading from a blood glucose meter (BGM), data from a continuous glucose monitor (CGM), data from a flash glucose monitor, blood glucose trend data, Insulin on Board (IOB) data, Carbohydrates on Board (COB) data, whether the PWD is or plans to exercise, whether the PWD is sick, whether the PWD is pregnant, whether the PWD is experiencing menses, and/or whether the PWD has consumed certain medications.

### BRIEF DESCRIPTION OF THE DRAWINGS

Example embodiments will be described and explained with additional specificity and detail through the use of the accompanying drawings in which:
FIG. 1 provides an example graph illustrating recorded meal sizes for boluses;
FIG. 2 provides an example graph illustrating percentages of users that rounded meal sizes for boluses;
FIG. 3 provides an example graph illustrating a relationship between an initial basal rate and a meal group size;
FIG. 4A illustrates an example interface for inputting a bolus;
FIG. 4B illustrates another example interface for inputting a bolus;
FIG. 4C illustrates another example interface for inputting a bolus;
FIG. 4D illustrates an example interface for initiating a bolus; and
FIG. 4E illustrates an example interface while a bolus is being delivered.

### DISCLOSURE

Devices, systems, and methods provided herein are adapted to reduce the cognitive burden on a user seeking to administer insulin for a meal while improving the accuracy of insulin bolus recommendations. In some cases, devices, systems, and methods provided herein can provide a plurality of preset meal sizes represented as icons or buttons for the user to select, which can represent the size of a typical meal for the user, a larger meal for the user, or a small meal for the user (and optionally tiny meals or extra-large meals for the user). In some cases, the number of carbohydrates assigned to each preset icon or button can be personalized for the user based on other user-specific dosage parameters entered by the user for an insulin delivery system (e.g., total daily basal dosage of insulin (e.g., U/day), a total daily dose of insulin, a carbohydrate ratio, an insulin sensitivity factor, a glucose setpoint, or a combination thereof). In some cases, the number of carbohydrates assigned to each preset icon or button can be personalized over time based on estimations of the size of each meal consumed when that icon or button is selected based on a glucose response after the consumption of each meal. In some cases, the number of carbohydrates assigned to each preset icon or button can be rounded to the nearest 5 grams of carbohydrates. In some cases, a user may manually enter personalized meal sizes for a number of user selectable icons or buttons. In some cases, a display can include the number of carbohydrates assigned to each preset icon or button. In some cases, after a user selects an icon, the user can select another user-selectable icon or button to deliver the recommended insulin bolus dose. In some cases, the user can modify the recommended insulin bolus dose prior to administering the bolus dose and select another user-selectable icon or button to deliver the altered insulin bolus dose. In some cases, the user can manually input a number of carbohydrates for a specific meal, modify number of carbohydrates assigned to a user-selectable icon or button. In some cases, a bolus calculation can use blood glucose data (e.g., from a BGM or estimated from a CGM) to adjust the bolus calculation to account for an elevated or low blood glucose level, which can optionally be displayed as a separate calculation separate from the calculation of the part of the bolus that is due to the consumption of carbohydrates. In some cases, methods, systems, and devices provided herein can use glucose data (e.g., from a continuous glucose monitor or flash glucose monitor) to automate the delivery of basal insulin.

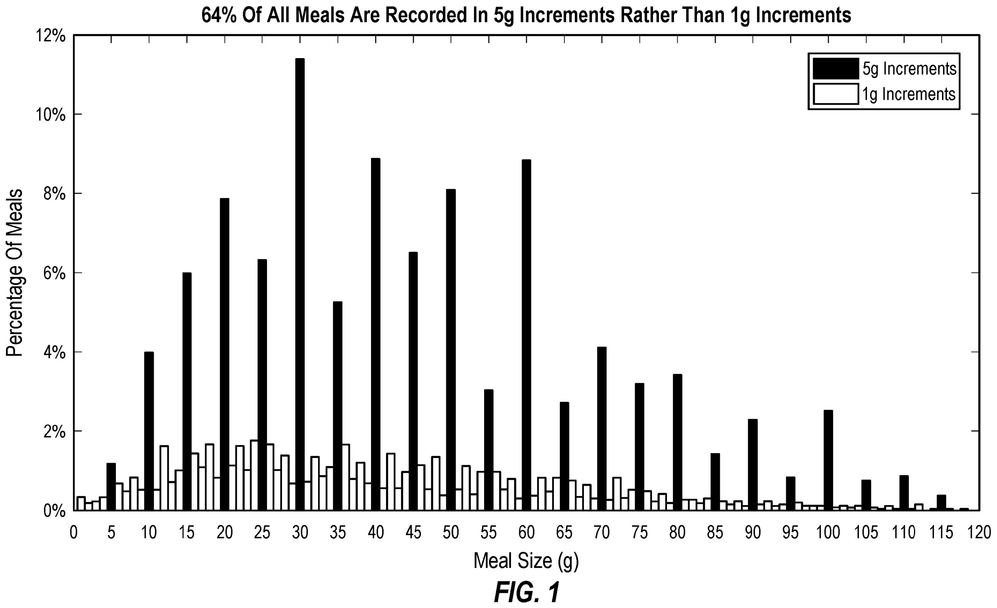

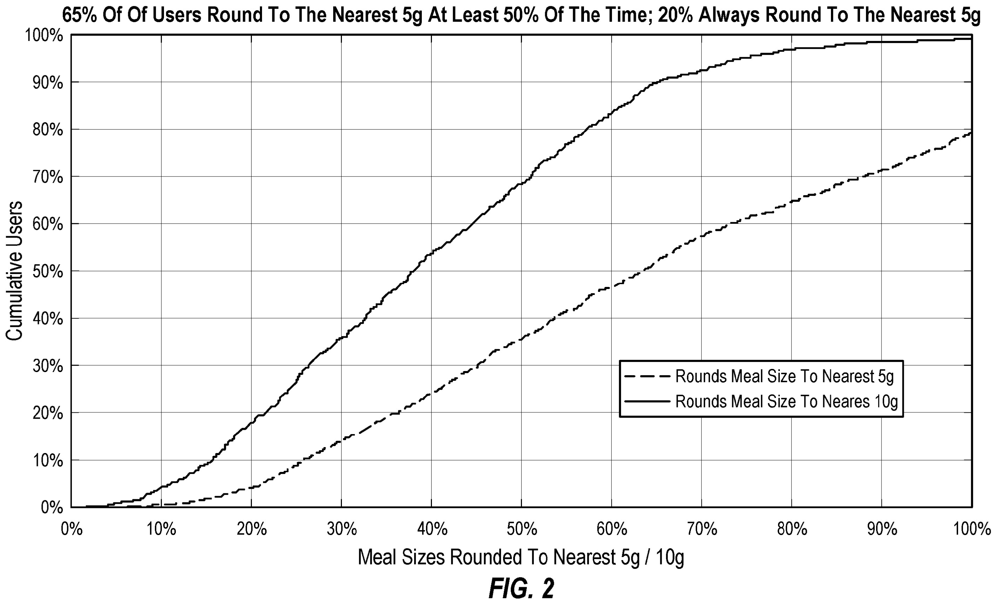

A review of real user meal inputs from users of an insulin delivery system including a bolus calculator can reveal that users typically enter meal sizes in increments of 5 grams. This bias towards increments of 5 grams reveals that many users of bolus calculators do not feel confident in their ability to accurately calculate their carbohydrate consumption down to 1 gram increments. A study of 969 subjects having 245,397 meal inputs revealed that 64% of those meals were recorded in 5 g increments, as depicted in FIG. 1. This data set also revealed that 65% of users rounded to the nearest 5 g at least 50% of the time and 20% always rounded to the nearest 5 g, as shown in FIG. 2.

Moreover, even if more users felt comfortable in attempting to make more specific estimations of the carbohydrate content of the foods that they select to eat, there is little data to suggest that these users would typically be within 5 grams of the actual carbohydrate count. For example, *"*Can children with type 1 diabetes and their caregivers estimate the carbohydrate content of meals and snacks?" C.E. Smart, K. Ross, J.A. Edge, B.R. King, P. McElduff, and C.E. Collins, Diabetic Medicine, 27, No. 3 (2010) pp. 38-353 illustrates the range or error there is in children having type 1 diabetes estimating the number of carbohydrates in meals and snacks.

Devices, systems, and methods provided herein can in some cases include user-selectable icons or buttons that describe (or represent) the size of a meal (e.g., in grams) in more subjective terms. In some cases, a user-selectable icon can be labeled as a medium sized meal or as a typical meal. In some cases, a user-selectable icon can be labeled as a small meal, a smaller meal, or a tiny meal. In some cases, a user-selectable icon can be labeled as a large meal, a larger meal, or an extra-large meal. In some cases, a user interface for bolus calculations can include a plurality of user-selectable icons or buttons that are each programmed with an amount of carbohydrates that are personalized for the subjective interpretation for that user. For example, some users might have a typical or medium sized meal of 50 grams while others might have a typical or medium sized meal of 20 grams. Additionally, users might have different subjective interpretations of what is a small meal, a medium meal, and a large meal for that specific user. Accordingly, by personalizing the sizes of meals for that specific user such that it matches the user's subjective understandings, devices, systems, and methods provided herein can achieve better bolus dose recommendations without burdening the user to try to estimate a number of carbohydrates for that particular meal.

The personalizing of one or more meal size user-selectable icons or buttons can be achieved using any suitable method. In some cases, a number of carbohydrates assigned to each user-selectable icon or button can be initially set at a predetermined starting point or can be determined based on entered user information, and then iteratively adjusted upward or downward based upon the glycemic response to that selected meal size and bolus over time.

Initial settings for one or more user-selectable icons or buttons included on a device or in a system provided herein can be preset to predetermined values or ranges (e.g., small = 20 g or 15-25g, medium = 30 g or 30-45g, large = 50 g or 50-75g, and extra-large = 80 g or 80-100g) or can be set based on entered user data or based on one or more user-specific dosage parameters entered into a device or system provided herein. In some cases, initial settings for the one or more user-selectable icons or buttons can be based on an initially entered or determined and programmed total daily basal dose (TDBD) / basal rate (e.g., U/day).

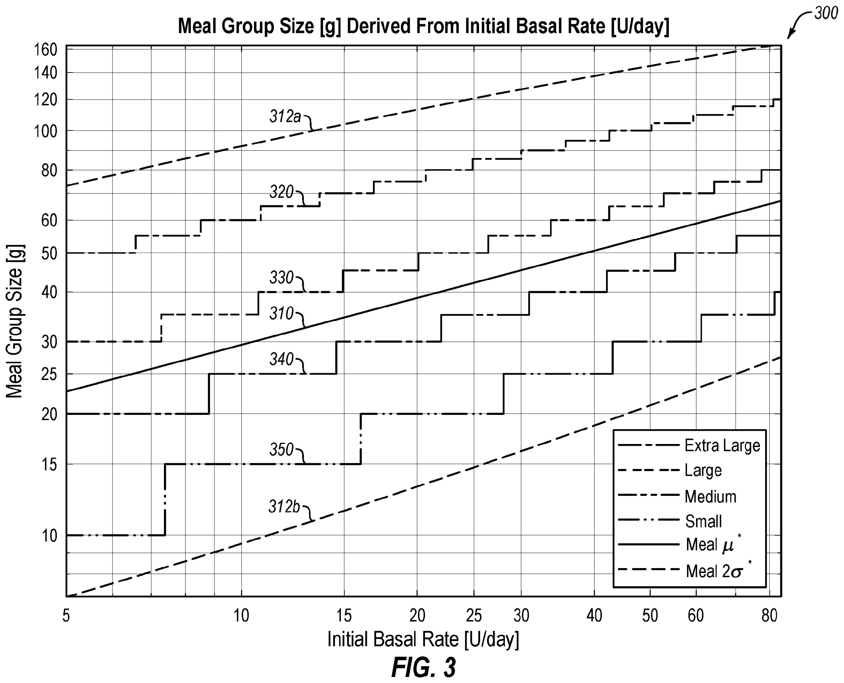

FIG. 3 depicts a chart illustrating example settings for 4 user-selectable icons or buttons based on an initially entered or determined TDB rate. In some cases, the preset values can be based on the Insulin Sensitivity Factor (ISF), Carb Ratio (CR), body weight, age, TDBD, and/or a combination thereof of the person with diabetes (PWD).

FIG. 3 illustrates the relationship between Basal Rates *(BR)* in units of [U/day] and Geometric Mean Meal Size µ*ᵤs in units of [g] (illustrated as 310 in FIG. 3) as characterized by the line corresponding to the major axis of the hyperellipsoid: *µ**_{MS} =12.1 ** BR*^{0.387}. The relationship between the Geometric Mean Meal Size and Geometric Standard Deviation Meal Size (*σ**_{MS}) is: *σ*⁺_{MS}=1.92- *µ**_{MS} / 186. FIG. 3 includes the Geometric Mean Meal Size as 310 and two times the Geometric Standard Deviation Meal Size as 312a and 312b. Accordingly, FIG. 3 illustrates initial meal size groups as corresponding to the 10% (as a small meal 350), 35% (as a medium meal 340), 65% (as a large meal 330), and 90% percentiles (as an extra large meal 320) of the Meal Size distribution by combining the above equations and rounding meal size groups to the nearest 5 grams. Thus, by way of example, for a user who had an initial basal rate of 10 U/day, the initial setting for the small meal size button or icon may correspond to 15 g (observed by the intersection of the small meal 350 with the initial basal rate of 10 U/day), the initial setting for the medium meal size button or icon may correspond to 25 g (observed by the intersection of the medium meal 340 with the initial basal rate of 10 U/day), the initial setting for the large meal size button or icon may correspond to 35 g (observed by the intersection of the large meal 330 with the initial basal rate of 10 U/day), and the initial setting for the extra large meal size button or icon may correspond to 60 g (observed by the intersection of the extra large meal 320 with the initial basal rate of 10 U/day).

In some cases, the relationship between typical meal sizes and other user-specific dosage parameters can be determined according to population statistics. For example, using the study of 969 subjects and their entered meal sizes discussed above, the distribution of log transformed Basal Rate *(BR)* in units of [U/day], Carb Ratio *(CR)* in units of [g/U], Insulin Sensitivity Factor (*ISF*) in units of [mg/dl/U], and meal size (MS) in units of [g] can be represented using the following multivariable normal, with mean and covariance matrices µ and Σ: $x ∼ N \left(μ, Σ\right) ; μ = \left[\begin{matrix}{μ}_{ln \left(BR\right)} \\ {μ}_{ln \left(CR\right)} \\ {μ}_{ln \left(ISF\right)} \\ {μ}_{ln \left(MS\right)}\end{matrix}\right] ;$ $\begin{matrix}Σ = \\ \left[\begin{matrix}{{σ}^{2}}_{ln \left(BR\right)} & {σ}_{ln \left(BR\right) , ln \left(CR\right)} & {σ}_{ln \left(BR\right) , ln \left(ISF\right)} & {σ}_{ln \left(BR\right) , ln \left(MR\right)} \\ {σ}_{ln \left(BR\right) , ln \left(CR\right)} & {{σ}^{2}}_{ln \left(CR\right)} & {σ}_{ln \left(CR\right) , ln \left(ISF\right)} & {σ}_{ln \left(CR\right) , ln \left(MR\right)} \\ {σ}_{ln \left(BR\right) , ln \left(ISF\right)} & {σ}_{ln \left(CR\right) , ln \left(ISF\right)} & {{σ}^{2}}_{ln \left(ISF\right)} & {σ}_{ln \left(MR\right) , ln \left(ISF\right)} \\ {σ}_{ln \left(BR\right) , ln \left(MR\right)} & {σ}_{ln \left(CR\right) , ln \left(MR\right)} & {σ}_{ln \left(MR\right) , ln \left(ISF\right)} & {{σ}^{2}}_{ln \left(MS\right)}\end{matrix}\right]\end{matrix}$

The mean and covariance matrices computed using robust statistics, with corresponding µ*, σ*, and correlation matrices ρ are $μ = \left[\begin{matrix}3.0111 \\ 2.3757 \\ 3.8645 \\ 3.6622\end{matrix}\right] ; Σ = \left[\begin{matrix}0.2843 & - 0.1657 & - 0.2216 & 0.0855 \\ - 0.1657 & 0.1978 & 0.1863 & - 0.0412 \\ - 0.2216 & 0.1863 & 0.2968 & - 0.082 \\ 0.0855 & - 0.0412 & - 0.082 & 0.1532\end{matrix}\right] ;$ $μ * = \left[\begin{matrix}20.3 U / day \\ 10.8 g / U \\ 47.7 \frac{mg}{dl} / U \\ 38.9 g\end{matrix}\right] ; σ * = \left[\begin{matrix}1.70 \\ 1.56 \\ 1.72 \\ 1.48\end{matrix}\right] ; ρ = \left[\begin{matrix}1 & - .70 & - 0.76 & 0.41 \\ - 0.70 & 1 & 0.77 & - 0.24 \\ - 0.76 & 0.77 & 1 & - 0.38 \\ 0.41 & - 0.24 & - 0.38 & 1\end{matrix}\right]$

Using these equations and rounding to the nearest 5 grams of carbohydrates, the chart of FIG. 3 can be obtained, which shows the distribution σ for meal sizes. It is contemplated, however, that additional population studies can be conducted to create better and/or different correlations between the typical, large, medium, and small meal sizes for a PWD based on their TBD, weight, age, ISF, CR, or a combination thereof.

In some cases, the number of carbohydrates associated with each user-selectable icon or button can be displayed on and/or adjacent to the user-selectable icon or button, which can help a user understand how to use the insulin delivery device or system to avoid deskilling the user. For example, seeing the number of carbohydrates assumed for each meal size helps a user that thinks about meals in terms of carbohydrates to adjust to using buttons to indicate a size of a meal. Additionally, by starting with display numbers rounded to the nearest 5 grams, the user can perceive that precision is not required, thus also reducing the cognitive burden on the user. Additionally, as the system iterates to personalize the amount of carbohydrates for each particular user-selectable icon or button, the system can adjust these numbers by smaller units (e.g., by 1 gram) to demonstrate to the user that the system is adjusting the number of carbohydrates associated with user-selectable icon or button.

Methods, systems, and devices provided herein can update the number of carbohydrates associated with each user-selectable icon or button using any suitable method. In some cases, methods, systems, and devices can use postprandial blood glucose data (e.g., between 1 hour and 3 hours after an announced meal) to evaluate whether the PWD likely consumed significantly more or significantly less carbohydrates than programmed for the user-selectable icon or button (e.g., +/-10%, +/-15%, +/-20%, etc. consumer carbohydrates). In some cases, one or more postprandial blood glucose thresholds can be used to evaluate the match between the amount of carbohydrates consumed and the amount of carbohydrates associated with a selected user-selectable meal icon or button. For example, methods, devices, and systems provided herein can ask a user for a postprandial blood glucose reading from a blood glucose meter. In some cases, methods, devices, and systems provided herein can receive postprandial blood glucose data from a continuous glucose monitor or flash glucose monitor. In some cases, methods, systems, and devices provided herein can use a single postprandial blood glucose data point and compare that to one or more upper thresholds and one or more lower thresholds for that period of time to determine whether the number of carbohydrates associated with that user-selectable meal icon or button should be adjusted upward or downward. For example, if a user selects a typical meal icon indicating a meal of 30 grams of carbohydrates, but the 2-hour postprandial blood glucose reading is above 200 mg/dL, the number of grams associated with that icon or button might be adjusted upward by 2 grams, if it is above 170 mg/dL, it might be adjusted upward by 1 gram, if it is below 130 mg/dL, it might be reduced by 1 gram, and if it is below 100 mg/dL, it might be reduced by 2 grams. Accordingly, over time the meal icons may be adjusted to more closely resemble the user's typical consumption patterns. The particular thresholds can be determined based on the postprandial time, the number of grams associated with the meal icon or button, the CR, ISF, and TDB, and setpoint of the PWD, etc.

In some cases, methods, systems, and devices provided herein can additionally automate insulin delivery using blood glucose data. Methods, systems, and devices provided herein can use any suitable algorithm to automate insulin delivery. In some cases, the blood glucose data can be from a continuous glucose monitor or flash glucose monitor. In some cases, methods, systems, and devices provided herein can deliver basal insulin doses throughout the day that vary based on the recent blood glucose data (e.g., within the last hour), estimations of the amount of active insulin in the PWD's body (e.g., Insulin On Board (IOB), estimations of the amount of active carbohydrates (e.g., carbohydrates on board), an Insulin Sensitivity Factor (ISF), a Carbohydrate-to-Insulin Ratio (CR), and other user-specific dosage parameters. In some cases, methods, devices, and systems provided herein can be a proportional-integral-derivative (PID) controller. In some cases, methods, devices, and systems provided herein can predict future blood glucose levels for different insulin delivery amounts or schedules and can pick an insulin delivery amount or schedule that minimizes a variation from a set point or that reduces the risk of future blood glucose levels exiting a predetermined range of blood glucose levels. An example of a suitable control algorithm is described in US Application No. 15/601,282, which is incorporated by reference herein in its entirety.

Methods, devices, and systems provided herein can use any suitable insulin delivery device or artificial pancreas system or closed loop insulin delivery system. In some cases, the insulin delivery device can be an insulin pump. In some cases, the insulin delivery device can be an insulin pump having a controller adapted to automate basal insulin delivery based on communications from a CGM or flash glucose monitor, such as described in US Application No. 15/601,282. Additionally or alternatively, the basal insulin delivery may be based on communication from a flash glucose monitor. In some cases, the insulin delivery device can be a smart insulin pen.

Methods, devices, and systems provided herein can in some cases have a user interface on a remote controller device (e.g., a smartphone) as described in US Application No. 15/601,282. In some cases, a remote controller device can wirelessly communicate (e.g., via Bluetooth Low Energy, Near-Field Communications, etc.) with an insulin delivery device (e.g., an insulin pump or a smart insulin pen) to send a request or instruction to the insulin delivery device regarding the information needed to deliver the calculated amount of insulin bolus. In some cases, methods, systems, and devices provided herein can include an insulin pump as the insulin delivery device and allow a user to deliver a bolus without the user directly accessing the insulin pump. In some cases, methods, systems, and devices provided herein can require a user to confirm a bolus delivery on an insulin pump before the bolus is delivered. In some cases, methods, systems, and devices provided herein may send a setting or instruction to a smart insulin pen (with or without allowing the user to alter the setting), but require that the user use the smart insulin pen to actually deliver each bolus.

A user interface on a remote controller device can include the user-selectable meal icons or buttons described above. In some cases, a remote controller device can be a wrist watch. In some cases, methods, devices, and systems provided herein can, in some cases, include a user interface that is part of an insulin delivery device that includes the user-selectable icons or buttons described above. In some cases, the user interface having the user selectable icons or buttons described above can be included on other devices, such as a BGM, a CGM, a flash glucose monitor, an insulin pump, a smart insulin delivery pen, or any other device associated with a PWD.

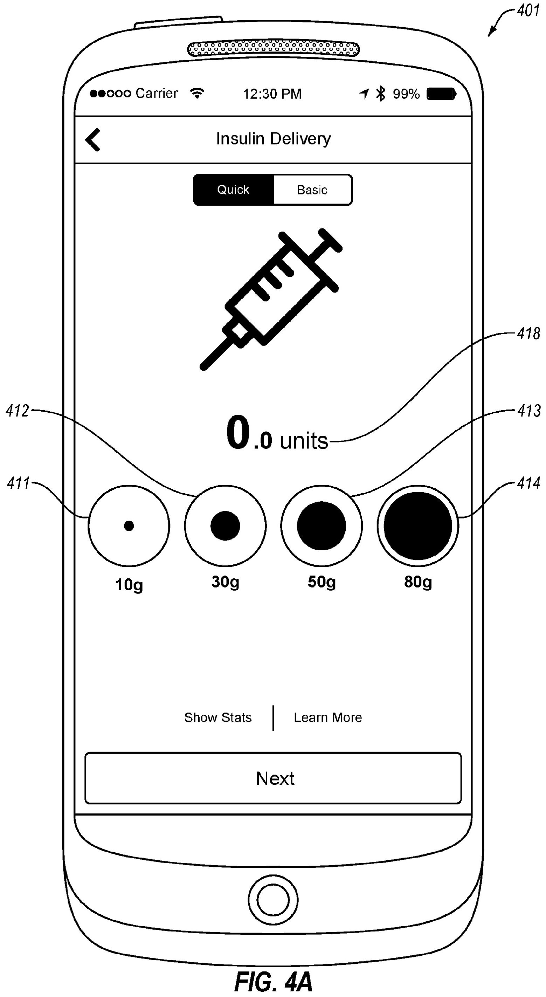

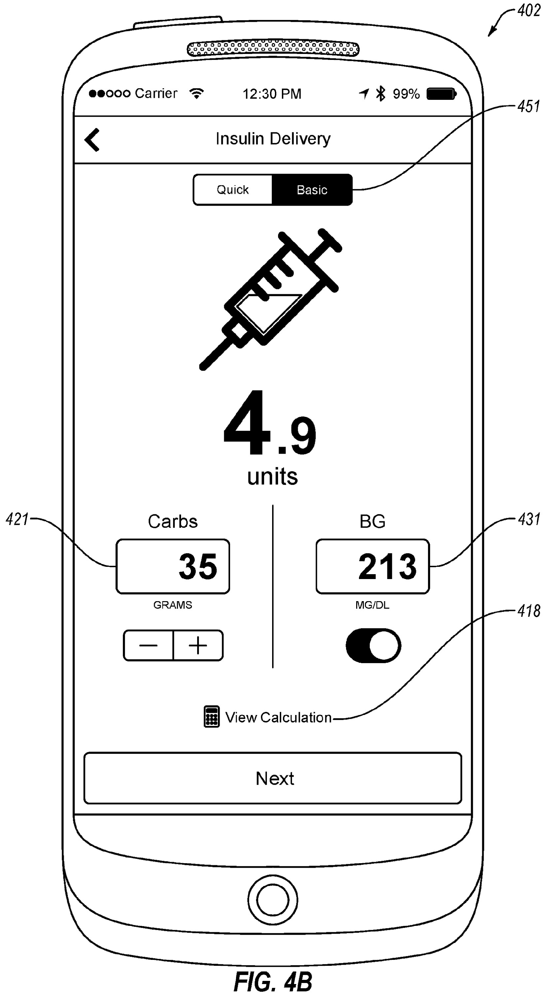

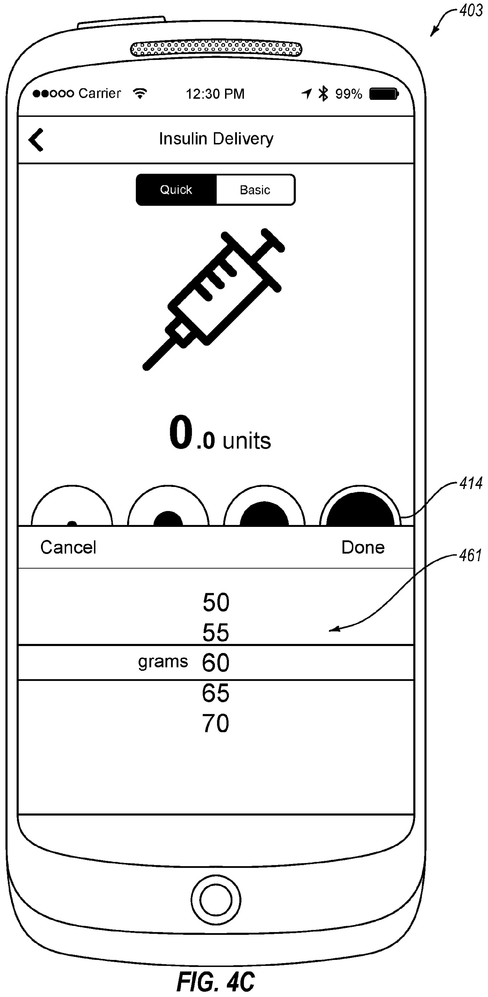

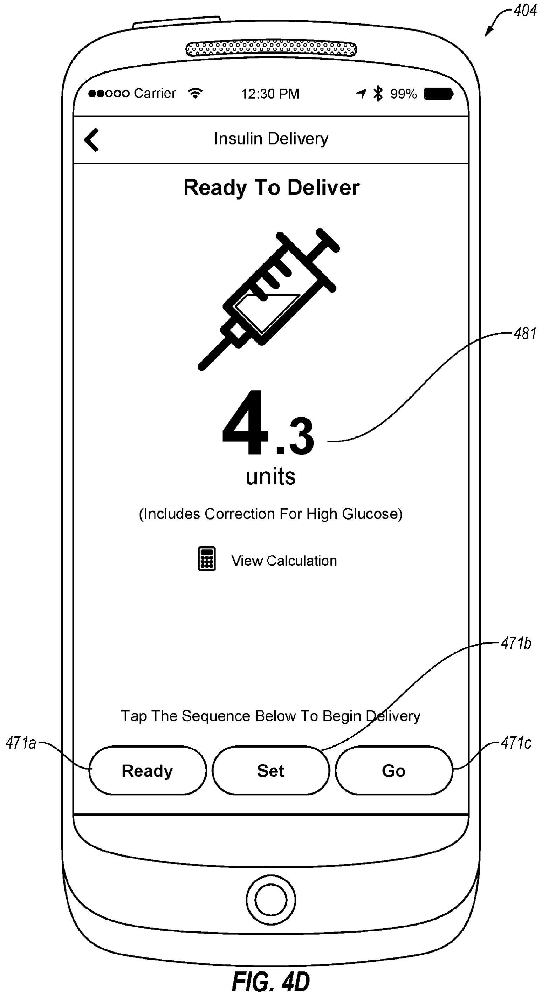

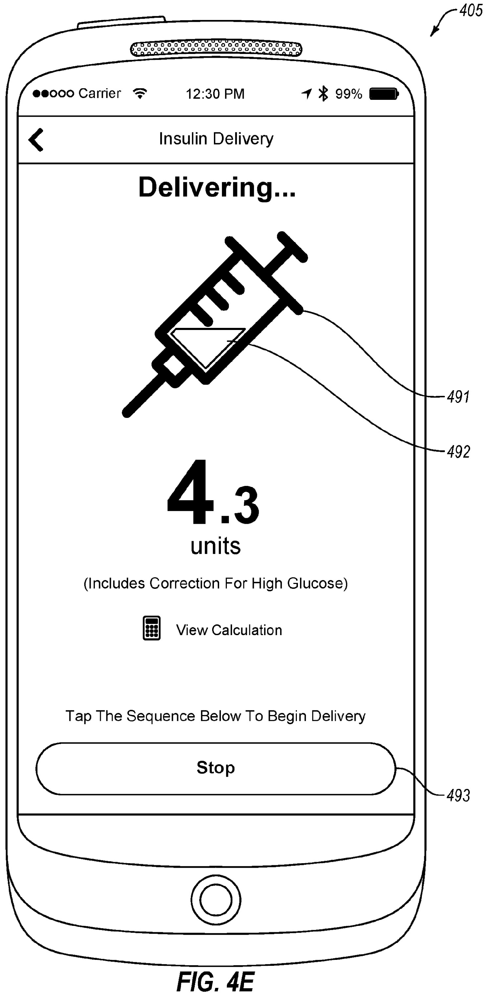

FIGS. 4A through 4E illustrate various example interfaces in accordance with the present disclosure. For example, FIG. 4A illustrates an example interface for inputting a bolus; FIG. 4B illustrates another example interface for inputting a bolus; FIG. 4C illustrates another example interface for inputting a bolus; FIG. 4D illustrates an example interface for initiating a bolus; and FIG. 4E illustrates an example interface while a bolus is being delivered.

As illustrated in FIGS. 4A and 4B, a user can toggle between a user interface 401 (of FIG. 4A) with a quick bolus screen having user selectable icons 411, 412, 413, and 414 that each represent a different number of carbohydrates (e.g., 10 g, 30 g, 50 g, and 80 g), and a user interface 402 (of FIG. 4B) with a basic bolus screen having a first field 421 that allow a user to enter grams of carbohydrates and/or a second field 431 to display or enter a blood glucose level, which may, for example, be received from a BGM, CGM, or flash glucose monitor. As discussed above, when first starting to use methods, systems, and devices described herein with a PWD, the settings of the selectable icons can be based on the user's TDB (e.g., as shown in FIG. 3) or other user-specific dosage parameters. Over time, the number of carbohydrates can be updated based on postprandial blood glucose data being over or under one or more thresholds. Although shown as 10 g, 30 g, 50 g, and 80 g in FIG. 4A, over time these numbers might eventually read, for example, as 8 g, 34 g, 47 g, and 85 g. In some cases, methods, systems, and devices provided herein may adjust adjacent user-selectable icon or button carbohydrate values to ensure a differentiation. For example, if the medium icon or button 112 is personalized up, the large icon or button 113 may also be adjusted upward to maintain a minimum differentiation between the amount of carbohydrates more than the medium button (e.g., at least 5 g, at least 10 g, or at least some other number of grams). In some cases, after selecting one of the icons 411, 412, 413, and/or 414, the user interface 401 may include a field 418 that may display a number of units of insulin to be delivered corresponding to the selected icon.

In some cases, the different number of carbohydrates associated with each of the user selectable icons 411, 412, 413, and/or 414 can be based on user-specific dosage parameters entered for the PWD when they first start the system. In some cases, the different number of carbohydrates associated with each user selectable icon can become personalized over time based on postprandial blood glucose data. In some cases, the different number of carbohydrates associated with each user selectable icon can vary based on the time of day (e.g., meal sizes might become personalized differently if the meal is in the morning vs. in the evening). In this example, each of the user selectable icons may include a graphic (e.g., a fork, a circle, a food item, or any other graphic) that has a size proportional to or related to the relative number of carbohydrates represented by the selectable icon. While four icons (411, 412, 413, and 414) are displayed, any number of icons may be used that represent different carbohydrate values. As discussed above the carbohydrate values represented by the icons may be predetermined and/or adjusted over time based on the user's postprandial blood glucose data. In some cases, an icon corresponding to a correction bolus only can be displayed.

FIG. 4B illustrates the user interface 402 where a user has toggled to a basic bolus screen where the user can enter a number of carbohydrates in the field 421 and optionally enter a blood glucose reading in the field 431. The user interface 402 can display the corresponding amount of insulin to be delivered for the entered number of carbohydrates and blood glucose level. In some cases, CGM data or flash glucose monitor data can be used in place of or in addition to data input in the field 431 corresponding to a blood glucose reading for the calculation of a bolus. In some cases, the user interface can indicate how much of a bolus is due to the consumed carbohydrates and how much is being administered to correct the current blood glucose level. In some cases, a user using user interfaces 401 and/or 402 of FIGS. 4A and 4B will be shown the number of units of insulin to be delivered (optionally explaining the calculation, which may include a correction component due to CGM data or flash glucose monitor data) before the user is allowed to issue the bolus command.

FIG. 4C illustrates another example interface 403 for inputting a bolus. For example, in some cases, a user might want to enter a more specific amount of carbohydrates, thus methods, systems, and devices provided herein can allow the user to enter a specific amount of carbohydrates. For example, as illustrated in FIG. 4C, an adjustable dial 461 may pop up in the interface 403 in response to a user pressing and holding an icon (e.g., the icon 414) rather than tapping the icon. A feature such as the adjustable dial may provide multiple carbohydrate values in 5 gram increments centered on the carbohydrate value represented by the icon. For example, if the icon represents 60 grams of carbohydrates then the associated pop up dial may include values greater than and less than 60 grams in 5 gram increments. In some cases, the 5 gram increments for each button are bound by the different number of carbohydrates associated with each of the adjacent user selectable icons. In some cases, a consistent selection of a different number of carbohydrates for a meal size can result in the number of carbohydrates associated with that user selectable icon updating to reflect a meal size typically selected by that user when using that icon (e.g., the meal size that is the median meal size, the meal size that is the median meal size, or the meal size that is the mode of meal sizes selected by that user). It may be that some users may select meal sizes in 5 gram increments, but other users may just quickly tap one of the icons (perhaps assuming that it is close enough and that adjustments to basal insulin rates will compensate for any discrepancy between the number of consumed carbohydrates and the number selected). In these and other embodiments, methods, devices, and systems of the present disclosure may provide functionality that services either approach to entering information to receive a bolus of insulin.

As noted above, the user device may calculate an insulin bolus dose corresponding to the selected carbohydrate amount. The insulin bolus dose may include a meal component (e.g., the number of carbohydrates divided by the PWD's carbohydrate-to-insulin ratio) and a correction component (e.g., the delta between the current blood glucose level and a target blood glucose level divided by the PWD's insulin sensitivity factor). In some cases, the calculation of a correction component may require a recent (e.g., within 15 minutes) reading from a blood glucose meter (BGM). In some cases, a correction component may be based on data from a continuous glucose monitor (CGM), a flash glucose monitor, or any other sensor configured to provide blood glucose levels. In some cases, a bolus calculation may consider blood glucose trend data, Insulin on Board (IOB), and/or Carbohydrates on Board (COB). In some cases, the bolus calculation can consider certain physiological conditions for the PWD, such as whether the PWD is or plans to exercise, whether the PWD is sick, whether the PWD is pregnant, whether the PWD is experiencing menses, and/or whether the PWD has consumed certain medications.

FIG. 4D illustrates an example interface 404 for initiating a bolus. For example, the interface 404 may require the user to tap multiple buttons 417 (e.g., the buttons 417a, 417b, and/or 417c) prior to delivery of insulin. The tapping of multiple buttons in a certain pattern may mitigate against unintentional deliveries of insulin. In this example, the user interface displays icons with the words: "ready", "set", and "go". The second icon may be unselectable until the first icon has been selected. The third icon may be unselectable until the second icon has been selected. Once the three icons have been selected an insulin bolus dose may be delivered to the user, for example, via an insulin delivery pump. Once the three icons have been selected, the application executing on the user device may send a message to an insulin delivery pump, for example, via a wireless communication device (such as an antenna), and/or chipset (such as a Bluetooth device (e.g., Bluetooth Low Energy, Classic Bluetooth, etc.), a Near-field communication (NFC) device, an 802.6 device (e.g., Metropolitan Area Network (MAN), a Zigbee device, etc.), a WiFi device, a WiMax device, cellular communication facilities, etc.), and/or the like, that includes the amount of insulin to be delivered to the user. The requirement to tap multiple buttons may reduce the chances that insulin is inadvertently delivered to the user. The interface 404 may also include a field 481 that displays the number of units of insulin to be delivered in the bolus. Additionally or alternatively, the interface 404 may indicate whether or not the bolus includes a correction portion of the bolus and/or what portion of the bolus is for correction purposes (e.g., to address a high blood glucose level).

In some cases, the order of the buttons 417 may be rearranged each time the user delivers or a bolus, or on any other regular interval. For example, the first time a PWD delivers a bolus in the day the buttons 417 may be in the order from left to right of 417a (Ready), 417b (Set), 417c (Go); a second time in the day delivering a bolus, the buttons 417 may be in the order from left to right of 417c (Go), 417b (Set), 417a (Ready), etc.

Fig. 4E illustrates an example interface 405 while a bolus is being delivered. In some cases, the interface 405 may include an indicator of the progression of delivery of insulin. For example, the insulin delivery screen may include an icon 491 that illustrates a syringe, with a decreasing amount of fluid 492. Additionally or alternatively, the indicator may include a decreasing numerical percentage, a colored circle that is filling/emptying or being drawn/erased, or any other display or visual indicator of progress of delivering insulin.

In some cases, the interface 405 may include a button or icon 493 that can be selected to stop the delivery of insulin. In some cases, invoking the button or icon 493 may trigger an alert or an alarm to the user that they interrupted the delivery of insulin and inquire whether or not they would like to resume or complete the delivery of the bolus of insulin.

In some cases, a completion screen may be displayed after an insulin bolus has been delivered. Additionally or alternatively, a screen may be displayed that illustrates an alert or an alarm that a blood glucose level is high and a correction bolus may be advisable for the user.

In some cases, a user may invoke an app icon displayed on a home screen of a user device such as, for example, a smart phone. The user can select the app icon via a touch screen or another user interface device, which results in execution of the corresponding application. In some cases, once executed on the user device, the application may include a home screen or landing screen. The home screen may display various data such as, for example, the user's current blood glucose data as received from a BGM, CGM, or flash glucose monitor. Various other data may be presented on the home screen, such as a calculated IOB, a blood glucose trend, and/or previous insulin delivery amounts and/or times. The home screen may also include a selectable button (or icon) that be tapped to allow the user to enter data regarding a meal bolus using a bolus screen. The invocation of such a meal bolus button or icon may lead the user to any of the screens of FIGS. 4A, 4B, 4C, etc.

The user interface described above can be used in an automated insulin delivery system adapted for use by a person having type 1 diabetes, but some aspects of this user interface can used by a person having Type 2 diabetes or gestational diabetes. In some cases, at least some aspects of the bolus user interface described above can be used in in a mobile application to personalize the treatment of Type 2 diabetes.

The embodiments described herein may include the use of a special-purpose or general-purpose computer including various computer hardware or software modules, as discussed in greater detail below.

Embodiments described herein may be implemented using computer-readable media for carrying or having computer-executable instructions or data structures stored thereon. Such computer-readable media may be any available media that may be accessed by a general-purpose or special-purpose computer. By way of example, and not limitation, such computer-readable media may include non-transitory computer-readable storage media including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices), or any other storage medium which may be used to carry or store desired program code in the form of computer-executable instructions or data structures and which may be accessed by a general-purpose or special-purpose computer. Combinations of the above may also be included within the scope of computer-readable media.

Computer-executable instructions comprise, for example, instructions and data which cause a general-purpose computer, special-purpose computer, or special-purpose processing device (e.g., one or more processors) to perform a certain function or group of functions. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

As used herein, the terms "module" or "component" may refer to specific hardware implementations configured to perform the operations of the module or component and/or software objects or software routines that may be stored on and/or executed by general-purpose hardware (e.g., computer-readable media, processing devices, etc.) of the computing system. In some embodiments, the different components, modules, engines, and services described herein may be implemented as objects or processes that execute on the computing system (e.g., as separate threads). While some of the system and methods described herein are generally described as being implemented in software (stored on and/or executed by general-purpose hardware), specific hardware implementations or a combination of software and specific hardware implementations are also possible and contemplated. In the present description, a "computing entity" may be any computing system as previously defined herein, or any module or combination of modulates running on a computing system.

Any ranges expressed herein (including in the claims) are considered to be given their broadest possible interpretation. For example, unless explicitly mentioned otherwise, ranges are to include their end points (e.g., a range of "between X and Y" would include X and Y). Additionally, ranges described using the terms "approximately" or "about" are to be understood to be given their broadest meaning consistent with the understanding of those skilled in the art. Additionally, the term approximately includes anything within 10%, or 5%, or within manufacturing or typical tolerances.

All examples and conditional language recited herein are intended for pedagogical objects to aid the reader in understanding the disclosure and the concepts contributed by the inventor to furthering the art, and are to be construed as being without limitation to such specifically recited examples and conditions. Although embodiments of the present disclosure have been described in detail, it should be understood that the various changes, substitutions, and alterations could be made hereto without departing from the spirit and scope of the disclosure.
Embodiments of the invention are described in the clauses below:
Clause 1. An insulin delivery system comprising:
   an insulin delivery device adapted to receive insulin and deliver insulin subcutaneously;
   a user interface in communication with the insulin delivery device and adapted to send bolus insulin instructions to the insulin delivery device, the user interface comprising a plurality of user-selectable icons or buttons each representing an amount of carbohydrates;
   memory to store one or more user-specific dosage parameter; and
   a processor in communication with the memory and adapted to receive blood glucose data, the processor being adapted to determine the amount of carbohydrates associated with each of the plurality of user-selectable icons or buttons based on at least one of the user-specific dosage parameters, the processor further being adapted to update the amount of carbohydrates associated with each of the plurality of user-selectable icons or buttons based upon the blood glucose data.
Clause 2. The insulin delivery system according to clause 1, wherein the plurality of user-selectable icons or buttons each represent an amount of carbohydrates in 5 gram or 10 gram increments.
Clause 3. The insulin delivery system according to clause 1, wherein the amount of carbohydrates represented by each of the plurality of icons is determined based on an insulin Sensitivity Factor (ISF), a Carb Ratio (CR), a body weight, an age, a total daily basal (TDB) rate, and/or a combination thereof of a person with diabetes (PWD).
Clause 4. The insulin delivery system according to clause 1, wherein the processor is further configured to determine an insulin delivery amount based on an amount of carbohydrates associated with a selected one of the user selectable icons or buttons and/or the blood glucose data.
Clause 5. The insulin delivery system according to clause 1, wherein the plurality of user-selectable icons or buttons each represent an amount of carbohydrates rounded to the nearest 5 grams.
Clause 6. The insulin delivery system according to clause 1, further comprising a glucose monitor adapted to monitor a glucose level of a person with diabetes and provide the blood glucose data to the processor.
Clause 7. The insulin delivery system according to clause 1, wherein the amount of carbohydrates associated with each of the plurality of user-selectable icons is determined from postprandial blood glucose data.
Clause 8. A method comprising:
   displaying at least three icons on a user interface of a mobile device, a first icon associated with the at least three icons associated with a first carbohydrate level, a second icon associated with the at least three icons associated with a second carbohydrate level, and a third icon associated with the at least three icons associated with a third carbohydrate level;
   receiving a user selection of one of the three icons through the user interface of the mobile device;
   determining an insulin bolus level from the user selection; and
   communicating the insulin bolus level to an insulin delivery device.
Clause 9. The method according to clause 8, further comprising receiving blood glucose data; wherein the insulin bolus level is determined in part from the blood glucose data.
Clause 10. The method according to clause 8, wherein adjusting the first carbohydrate level, the second carbohydrate level, and the third carbohydrate level based on an insulin Sensitivity Factor (ISF), a Carb Ratio (CR), a body weight, an age, a total daily basal (TDB) rate, and/or a combination thereof of a person with diabetes (PWD).
Clause 11. The method according to clause 8, wherein the insulin bolus level is communicated to the insulin delivery device in response to a user selection indicating delivery of insulin, wherein the user selection includes a fail safe procedure.
Clause 12. The method according to clause 8, wherein the insulin bolus level is communicated to the insulin delivery device in response to a user selection indicating delivery of insulin, wherein the user selection includes a plurality of taps or gestures from a user.
Clause 13. The method according to clause 8, wherein the first carbohydrate level is rounded to the nearest 5 grams, the second carbohydrate level is rounded to the nearest 5 grams, and the third carbohydrate level is rounded to the nearest 5 grams.
Clause 14. The method according to clause 8, further comprising: receiving postprandial blood glucose data; and adjusting at least one of the first carbohydrate level, the second carbohydrate level, and the third carbohydrate level based on the postprandial blood glucose data.
Clause 15. The method according to clause 8, wherein the insulin bolus level is determined from factors selected from a list consisting of: a number of carbohydrates divided by the PWD' s carbohydrate-to-insulin ratio, a difference between a current blood glucose level and a target blood glucose level divided by the PWD's insulin sensitivity factor, a reading from a blood glucose meter (BGM), data from a continuous glucose monitor (CGM), data from a flash glucose monitor, blood glucose trend data, Insulin on Board (IOB) data, Carbohydrates on Board (COB) data, whether the PWD is or plans to exercise, whether the PWD is sick, whether the PWD is pregnant, whether the PWD is experiencing menses, and whether the PWD has consumed certain medications.
Clause 16. One or more non-transitory, computer-readable media containing instructions that, in response to being executed by one or more processors, cause a mobile device to perform operations comprising:
   display at least three icons on a user interface of the mobile device, a first icon associated with the at least three icons associated with a first carbohydrate level, a second icon associated with the at least three icons associated with a second carbohydrate level, and a third icon associated with the at least three icons associated with a third carbohydrate level;
   receive a user selection of one of the three icons through the user interface of the mobile device;
   determine an insulin bolus level from the user selection; and
   communicate the insulin bolus level to an insulin delivery device.
Clause 17. The computer-readable media of clause 16, wherein the insulin bolus level is communicated to the insulin delivery device in response to a user selection indicating delivery of insulin, wherein the user selection includes a fail safe procedure.
Clause 18. The computer-readable media of clause 16, wherein the insulin bolus level is communicated to the insulin delivery device in response to a user selection indicating delivery of insulin, wherein the user selection includes a plurality of taps or gestures from a user.
Clause 19. The computer-readable media of clause 16, wherein the first carbohydrate level is rounded to the nearest 5 grams, the second carbohydrate level is rounded to the nearest 5 grams, and the third carbohydrate level is rounded to the nearest 5 grams.
Clause 20. The computer-readable media of clause 16, wherein the operations further comprise:
   receive postprandial blood glucose data; and
   adjust at least one of the first carbohydrate level, the second carbohydrate level, and the third carbohydrate level based on the postprandial blood glucose data.

## Claims

1. A method for determining an insulin bolus dose, comprising:
displaying at least three icons on a user interface, wherein the first icon is associated with a first carbohydrate level, the second icon is associated with a second carbohydrate level, and the third icon is associated with a third carbohydrate level;
receiving a user selection of one of the three icons through the user interface;
displaying, on the user interface, means for adjusting a carbohydrate level associated with the user-selected icon;
receiving a user selection of an adjusted carbohydrate level associated with the user-selected icon through the means for adjusting the carbohydrate level
determining the insulin bolus dose based on the adjusted carbohydrate level associated with the user-selected icon; and
communicating the insulin bolus dose to an insulin delivery device.

2. The method of claim 1, wherein the means for adjusting the carbohydrate level comprises a user-adjustable dial.

3. The method of claim 1, wherein the means for adjusting the carbohydrate level is centered on the carbohydrate level associated with the user-selected icon.

4. The method of claim 1, wherein carbohydrate levels provided by the means for adjusting the carbohydrate level are bounded by carbohydrate levels associated with the icons adjacent to the user-selected icon.

5. The method of claim 1, wherein the means for adjusting the carbohydrate level provides carbohydrate levels adjustable in predetermined increments.

6. The method of claim 1, wherein the insulin delivery device comprises an insulin pump, and the user interface is on the insulin pump.

7. The method of claim 1, wherein the insulin bolus dose is a bolus dose to be delivered by the insulin delivery device in response to selection of icons on the user interface by the user.

8. The method of claim 1, wherein the user interface comprises an indicator of a progression of delivery of insulin.

9. The method of claim 1, wherein the user interface comprises an icon that can be selected to stop the delivery of insulin.

10. The method of claim 1, wherein carbohydrate levels provided by the means for adjusting the carbohydrate level are greater than and less than the carbohydrate level associated with the user-selected icon.

11. The method of claim 1, wherein the first, second, and third carbohydrate levels vary based on a time of day.

12. The method of claim 1, further comprising displaying, on the user interface, the insulin bolus dose, and wherein the insulin bolus dose is the bolus dose to be delivered by the insulin delivery device.

13. The method of claim 1, wherein the user interface does not display a numerical value associated with the first carbohydrate level, the second carbohydrate level, or the third carbohydrate level.

14. A non-transitory computer-readable medium storing instructions that, when executed by one or more processors, cause the one or more processors to execute the method as in any of claims 1-13.

15. An insulin pump system, comprising:
a user interface;
an insulin reservoir;
a pumping mechanism configured to deliver insulin from the insulin reservoir to a person with diabetes; and
one or more processors operatively coupled to the user interface and the pumping mechanism, the one or more processors configured to execute the method as in any of claims 1-13.
